# EUROPÄISCHE PATENTSCHRIFT

(11) **EP 2 812 479 B1**
(45) Veröffentlichungstag und Bekanntmachung des Hinweises auf die Patenterteilung: **20.07.2016**
(21) Anmeldenummer: 13706411.9
(22) Anmeldetag: 31.01.2013
(51) Int. Cl.: D06M 11/44, D06M 11/46, D06M 13/00, D06M 13/352, D06M 13/355, D06M 13/503, D06M 15/263, D06M 15/37, D06M 16/00, A01N 25/10, A01N 25/34, A01N 59/16, A01N 43/78, A01N 43/40, A47K 3/38

(54) **DUSCHVORHANG MIT ANTIBEWUCHS-AUSRÜSTUNG UND VERFAHREN ZU SEINER HERSTELLUNG**
SHOWER CURTAIN WITH ANTI-FOULING FACILITY AND METHOD FOR THE PRODUCTION THEREOF
RIDEAU DE DOUCHE À PROTECTION ANTI-SALISSURES ET SON PROCÉDÉ DE FABRICATION

(30) Priorität: 09.02.2012 DE 202012100443 U; 19.04.2012 DE 102012103438
(43) Veröffentlichungstag der Anmeldung: 17.12.2014
(73) Patentinhaber: Wenko-Wenselaar GmbH & Co. KG, 40721 Hilden (DE)
(72) Erfinder: KÖLLNER, Hanns-Joachim, 40625 Düsseldorf (DE)
(74) Vertreter: Kreuzkamp, Markus
(86) Internationale Anmeldenummer: PCT/DE2013/100034
(87) Internationale Veröffentlichungsnummer: WO 2013/117188

(56) Entgegenhaltungen:
- JP-A- H1 127 946
- US-A1- 2009 258 557
- DATABASE WPI Week 200165 Thomson Scientific, London, GB; AN 2001-573787 XP002712885, -& JP 2001 131874 A (TEIJIN LTD) 15. Mai 2001 (2001-05-15)
- DATABASE WPI Week 199606 Thomson Scientific, London, GB; AN 1996-056098 XP002712886, & JP H07 316336 A (DAINIPPON INK & CHEM INC) 5. Dezember 1995 (1995-12-05)

## Beschreibung

### Technischer Bereich

Die vorliegende Erfindung betrifft einen Duschvorhang gemäß dem Oberbegriff der unabhängigen Ansprüche.

Allgemeine Ansätze zur nass-chemischen Ausrüstung von textilen Trägern mit Antibewuchs-Ausrüstung finden sich in der US 2011 /0 217 348 A1. Dieses Dokument beansprucht die Verwendung so ausgerüsteter Träger im Bereich der Fischerei und Aquakultur-Netze. Problematisch ist hierbei jedoch, dass solche Netze dauerhaft dem Wasser ausgesetzt sind. Die Antibewuchs-Ausrüstung muss daher nachteilig nahezu wasserunlöslich sein, um nicht in kurzer Zeit durch das Seewasser ausgewaschen zu werden. Vor diesem Hintergrund stellt sich vorliegend die Frage, welches der in diesem Dokument allgemein offenbarten Ausrüstungs-Systeme für einen Duschvorhang, welcher nur periodisch mit Wasser in Kontakt kommt und von Feuchte-Schimmel befallen wird, überhaupt geeignet sein könnte. Ohne dauerhaften Wasserkontakt kann nicht von einer ausreichenden Aktivität der hier offenbarten Ausrüstungs-Systeme auf der entsprechend ausgerüsteten, textilen Oberfläche eines Duschvorhanges ausgegangen werden.

Als Alternative zu nass-chemischen Ausrüstungsverfahren offenbart die WO 2005 123 891 A2 ein Vakuum-Plasma-Verfahren, bei dem Bindungen bildende Komponenten mit inerten Komponenten vermischt und in einer Plasma-Vakuumkammer in molekular innig homogenisiertem Gemisch auf einem Reinigungs-Textil für den Haushaltsgebrauch abgeschieden werden. Zwar ist davon auszugehen, dass diese Textilien übereinstimmend nur zeitweilig zum Putzen/Reinigen befeuchtet werden, jedoch bedingt ein Plasma-Vakuumverfahren nachteilig hohen technischen Aufwand und hohe Fertigungskosten.

Eine physikochemische Beschichtung ist aus der US 2009/0258557 A1 bekannt: Ein Polyester-Faser-Textil wird vorgelegt, mit einer Ausrüstungsmischung auf Toluolsulfonsäurebasis benetzt und anschließend so stark erwärmt, dass für etwa 5 Minuten eine maximale Diffusion einer bewuchshemmenden Sulfonsäureverbindung zusammen mit einem zweiten Biozid in das textile Substrat erzielt wird. So speziell mit zwei Wirkstoffen in Kombination ausgerüstete Textilien sind industriell und/oder gastronomisch mehrfach reinigbar, ohne dass die fungizide Eigenschaft beeinträchtigt werden würde. Gleichsinnig beschreibt die JP 2001 131874 A ein mit feuerhemmenden Phosphorverbindungen copolymerisiertes Polyester-Gewebe, welches nachträglich mit halogenierten Cycloalkan-Verbindungen, Stickstoff- und/oder Sauerstoff -Verbindungen und wasserabweisenden Fluoropolymeren konditioniert wird. Ähnliche Flammschutz-Kompositionen sind auch aus dem Halbleiter-Bereich bekannt, wie zum Beispiel der JP H11 27946 A.
Gerade im privaten Bereich werden Duschvorhänge jedoch sehr viel länger verwendet als dies im industriellen oder gastronomischen Bereich akzeptabel wäre. Nachteilig ist somit, dass von der industriellen Reinigbarkeit, die durch kurze, harte Bedingungen und Lebenszyklen gekennzeichnet ist, nicht auf eine Langzeit-Stabilität einer bewuchshemmenden Ausrüstung geschlossen werden kann.

Eine nasschemische Beschichtung offenbart die WO 2008 051 756 A2: Eine thermoplastische, wässrige Harz-Mischung kann bei der Ausformung oder Extrusion von Glasfaserbasierten oder Glas-basierten Formlingen oder Geweben eine verbindende Matrix bereitstellen. Hier bleibt allerdings offen, ob und wie solch eine Matrix als Reservoir für eine Wirkstoff-Ausrüstung dienen könnte. Zudem stellt sich die Frage, welche Eigenschaften diese Matrix in einem Stoffgewebe, welches gar keine Glasfasern enthält und nicht geformt werden muss, aufweisen könnte. Gerade die letzte Frage ist hierbei relevant, da die etablierten Duschvorhänge keiner Glasfaser-Verstärkung bedürfen und auch keine Glaspartikel enthalten. Angesichts des allgemeinen, technischen Hintergrunds stellt sich zunächst die Frage, welche nasschemisch aufgebrachten Ausrüstungen eine funktionierende Antibewuchs-Ausrüstung für einen Duschvorhang bereitstellen können.

### Beschreibung des Standes der Technik

Gattungsgemäße Duschvorhänge weisen eine Antibewuchs-Ausrüstung auf, welche verhindern soll, dass der regelmässig durchnässte Duschvorhang gerade in den langsam trocknenden, seitlich an der Duschwanne oder Wanne anliegenden Bereichen von Keimen und/oder Schimmel befallen und optisch unansehnlich wird. Zudem wird so einer inakzeptablen Belastung mit pathogenen Schimmel- sowie Keim-Kolonien im Badbereich vorgebeugt.

Aus der DE 203 06 281 U1 ist ein Duschvorhang bekannt, welcher mit einer keimtötenden Lösung auf ionischer Basis behandelt worden ist.

Nachteilig ist bei den gattungsgemässen Duschvorhängen, dass die gegen den Befall mit Keimen und/oder Schimmel eingebrachten Wirkstoffe sowohl hoch wirksam als auch wasserlöslich sein müssen, damit die in wässriger Umgebung gedeihenden Keime auch erreicht und im Wachstum zumindest gehemmt werden können. Durch die notwendige Löslichkeit in Wasser gelangen die Wirkstoffe jedoch während des Duschens direkt in das Abwasser und in nachgeschaltete Kläranlagen und töten dort hoch wirksam die Klärschlamm-Bakterien. Zudem werden die Wirkstoffe dadurch schnell ausgewaschen und die angestrebte Langzeitwirkung der Wirkstoffe ist somit häufig nicht gewährleistet.

Aufgabe der vorliegenden Erfindung war es daher, die Nachteile des Standes der Technik zu überwinden und einen Duschvorhang mit Antibewuchs-Ausrüstung bereitzustellen, welcher die widersprüchlichen Anforderungen von zuverlässiger Wirksamkeit der keimtötenden Komponenten und der notwendigen Langzeitwirkung gleichermaßen zu erfüllen vermag.

Die Lösung dieser Aufgabe erfolgt gemäß der Merkmale der unabhängigen Ansprüche.

Vorteilhafte Ausführungsformen ergeben sich aus den abhängigen Ansprüchen sowie der nachfolgenden Beschreibung.

### Zusammenfassung der Erfindung

Erfindungsgemäß weist ein Duschvorhang mit Antibewuchs-Ausrüstung ein auf seinem Gewebe verankertes, flüssiges Wasser abweisendes, offen vernetztes, mehrkomponentiges Polykondensat auf. Das Polykondensat umfasst mindestens die Teilkondensate der Ausgangsstoffe
a) Acrylat-Präpolymer
b) aliphatisches Diol und
c) Zink-organische Verbindung mit Hydroxylgruppe,
Rest Hilfsstoffe, Zusätze und unvermeidbare Verunreinigungen.

### Beschreibung der Erfindung und vorteilhafter Merkmale

Gewebe bezeichnet hierbei das Grundmaterial des Duschvorhangs, welches Baumwolle, Wolle, Kunststoff und/oder Mischungen der verschiedenen Materialsorten aufweist. Solch ein Gewebe kann vorteilhaft bedruckt sein, um den Duschvorhang optisch passend zu bestimmten Einrichtungs- und Designstilen auszugestalten.

Ein Polykondensat bezeichnet hierbei ein durch Kondensation - also durch Einengen unter Entzug der bei der Verknüpfung entstehenden Abspaltprodukte - aus verschiedenen Komponenten gewonnenes Gerüst / Polymer. Das Gerüst weist dann in seiner Struktur die verschiedenen Komponenten als eingebaute Teilelemente auf. Diese Teilelemente, welche die im Rahmen der Kondensation eingebundenen, zuvor freien Komponenten sind, werden nachfolgend als Teil des Gerüsts gleichsinnig als Teilkondensate bezeichnet. Eine Polykondensation unter Wasserabspaltung erscheint hierbei wenig vorteilhaft, da diese einer Rückreaktion unterliegen kann, wodurch das Polykondensat aufgelöst und frühzeitig von dem Duschvorhang entfernt werden könnte. Überraschender Weise haben die Erfinder festgestellt, dass ein Polykondensat auf Polyacrylatbasis kombiniert mit quervernetzenden, aliphatischen Verbindungen mit Hydroxyl-Funktionen ein Kondensat aus miteinander vernetzten und verbunden Komponenten bereitstellt, welches sowohl Stabilität des Kondensats als auch Langzeit-Wirksamkeit der Komponenten bereitstellt.

Das Polykondensat auf Polyacrylatbasis wird auf dem Gewebe verankert. Hierzu wird das Gewebe in ein - vorteilhaft präpolymerisiertes - Acrylat-Bad eingelegt, bis eine Durchdringung von mindestens 80 % Gewichtszunahme bezogen auf das Tropfgewicht eines völlig durchtränkten Gewebes erreicht wurde. Anschließend wird das Gewebe dem Bad entnommen und die Kondensation des Acrylat-Präpolymers zum vernetzten Polyacrylat durchgeführt, um ein flüssiges Wasser abweisendes Kondensat zumindest über die gesamte Außenfläche des Duschvorhangs aufzubringen.

Präpolymer bezeichnet hierbei die monomere oder oligomere Vorstufe des Polyacrylats; Acrylat-Monomere umfassen hierbei Acrylsäure-Moleküle mit C-C-Doppelbindungs- und Säurefunktion und Gemische von Acrylsäure-Molekülen mit Alkoholen mit einer Mehrzahl an OH-Gruppen und optional COOH-Gruppen. Oligomere Präpolymere sind anteilig vorvernetzte/vorkondensierte Makromoleküle, welche vorteilhaft als einheitliche, größere Bausteine des angestrebten Polykondensats nur noch abschließend auf dem Gewebe verankert und miteinander vernetzt werden müssen. Oligomere Präpolymere erlauben vorteilhaft eine genauere Kontrolle der Gleichmäßigkeit des Vernetzungsgrads des resultierenden Polykondensats. Besonders vorteilhaft werden hierbei hydrophobe Wirkstoffe in einem gaspermeabel aufgebauten, oligomeren Präpolymer geträgert in das Polykondensat mit eingebracht.

Die Erfinder gehen davon aus, dass durch die zusätzlichen Komponenten mit Hydroxylfunktion ein offen vernetztes, mehrkomponentiges Polykondensat erzielt wird, welches den Durchtritt von Wasserdampf zwar erlaubt, aber gegenüber direkt aufliegendem, flüssigen Wasser eine hydrophobe Sperrwirkung entfaltet. Das Polykondensat umfasst hierbei mindestens die Teilkondensate der Ausgangsstoffe a) Acrylat-Präpolymer, b) aliphatisches Diol und c) Zink-organische Verbindung mit Hydroxylgruppe. Während das Polyacrylat ein grundlegendes, über C-C-Brücken und/oder Esterbrücken aufgebautes Gerüst bereitstellt, vermag das Diol unter Wasserabgabe mit freien, organischen Säuregruppen weitere Esterbrücken des Typs R-CO-O-R' auszubilden und zusätzliche Vernetzungen in das Kondensat einzubringen. Die Zink-organische Verbindung mit Hydroxylgruppe kann über Wasserstoffbrückenbindungen, Ether-Brücken des Typs R-O-R' sowie Ester-Brücken des Typs R-CO-O-R' in das Kondensat eingebunden werden. Diese aus dem Bereich der Terpolymere bekannten, vielfältigen Verknüpfungs- und Assoziationsmöglichkeiten machen die Erfinder dafür verantwortlich, dass wider Erwarten ein diffusionsoffenes Polykondensat erhalten werden konnte. Wäre das Polykondensat nämlich geschlossen, so wären die keimtötenden Komponenten fest darin eingeschlossen und könnten keinerlei Wirkung entfalten. Die keimtötende Wirkung konnte aber in umfangreichen Versuchen nachgewiesen werden, obwohl in oberflächlich abperlendem Wasser die keimtötenden Komponenten nicht nachweisbar waren. Die Erfinder gehen davon aus, dass das Polykondensat offene Poren mit durchgängigen Kanälen aufweist, über welche die Komponenten gasförmig an die Außenfläche des Duschvorhangs gelangen und dort wirksam werden können. Diese Diffusion, welche durch gasförmiges Wasser welches die Komponenten aktivieren kann gefördert wird, wirkt bei feuchten Bedingungen einem Keimbefall wirksam entgegen, während unter trockenen Bedingungen die unter Wasserentzug im Polykondensat verankerten Wirkstoffe nicht freigesetzt werden können. Das vorbeschriebene Erklärungsmodell der Erfinder vermag sinnvoll darzulegen, wie die grundsätzlich wasserlöslichen Wirkstoffe über ein Polykondensat erstmals als Langzeitwirkstoff in einem Duschvorhang bereitgestellt werden konnten. Es versteht sich, dass der Duschvorhang Hilfsstoffe, Zusätze und unvermeidbare Verunreinigungen enthalten kann, solange die vorbeschriebene Wirksamkeit der keimtötenden Wirkstoffe erhalten bleibt.

Bevorzugt umfassen Hilfsstoffe Haftvermittler in Form von polymerisierbaren, besonders bevorzugt präpolymerisierten polymerisierbaren, Verbindungen, welche zuvor und/oder gleichzeitig mit dem Polykondensat eingebracht werden und haftvermittelnde, bevorzugt zusätzlich wasserdichte, Strukturen zwischen dem Gewebe und dem Polykondensat ausbilden. Bevorzugt umfassen Hilfsstoffe polymerisierbare Verbindungen, welche mit der Doppelbindung der Acrylat-Baugruppen im Polykondensat thermisch vernetzt, besonders bevorzugt im Rahmen eines abschließenden Aushärtens und Trocknens bei 60 bis 200°C, bevorzugt 70 bis 180°C, besonders bevorzugt 100 +- 40°C, stabilisierend mit in die Beschichtung eingebracht werden. Besonders bevorzugt weist der Duschvorhang eine jeweils verschieden ausgestaltete Innen- und Außenseite auf, wobei eine Seite mit der Antibewuchs-Ausrüstung ausgestattet ist; die einseitige Ausrüstung kann so gezielt in voreingestellten Raff-Bereichen, welche langsam trocknen und daher anfällig für Bewuchs sind, angebracht werden, während schnell trocknende, konvex gewölbte Bereiche / Außenbereiche durch das Gewebe hindurch mit einer geringeren Aktivität der bewuchshemmenden Wirkstoffe gleichfalls ausreichend geschützt sind; so wird bevorzugt mit einer asymmetrischen Anordnung und Ausgestaltung des Duschvorhangs vorteilhaft eine sehr gute Wirksamkeit bei reduzierter Gesamtmenge an Wirkstoff eingestellt.

Bevorzugt ist der Duschvorhang dadurch gekennzeichnet, dass aliphatische Diole der Gruppe b) eine längste, unverzweigte Kette von 4 bis 8 C-Atomen aufweisen. Durch eine Quervernetzung mit Diolen dieser Größe / Länge wird vorteilhaft eine gleichmäßigere Porenstruktur und Diffsuionsoffenheit des Polykondensats eingestellt.

Bevorzugt ist der Duschvorhang dadurch gekennzeichnet, dass das Polykondensat eine Benzimidazol-Verbindung als Zusatz aufweist. Benzimidazole haben je nach Struktur fungizide oder auch biozide/antimikrobielle Eigenschaften. Hierbei bedingt die aromatische Struktur hydrophobe Eigenschaften der Grundverbindung, d. h. diese Wirkstoffe sind wässrig aus einem Grundgerüst kaum auslösbar, wirken aber bei direktem Eindringen von Sporen oder Keimen einem Bewuchs im Inneren des Kondensats vorteilhaft entgegen. Besonders bevorzugt ist der Duschvorhang dadurch gekennzeichnet, dass dass das Polykondensat 2-(4-Thiazolyl-)-1 H-benzimidazol als Zusatz aufweist. Mit diesem Benzimidazol konnten besonders vorteilhaft wachstumshemmende und keimtötende Wirkungen gegenüber Pilzen und Keimen in Kombination erzielt werden.

Bevorzugt ist der Duschvorhang dadurch gekennzeichnet, dass das Polykondensat als Ausgangs-Diol der Gruppe b) 2-Methylpentan-2,4-diol als Diol aufweist. Durch die zusätzlichen Substituenten werden die OH-Gruppen dieses Diols sterisch stärker ausgerichtet; darauf führen die Erfinder zurück, dass unter Verwendung dieses Diols eine deutlich gleichmäßigere Beschichtung mit flächig gleich wirksamer, keimtötender Imprägnierung erzielt werden konnte. Die verbesserte Gleichmäßigkeit kann mit einer einheitlicheren Ausrichtung des Diols und daraus resultierenden, einheitlichen Porenstruktur im Polykondensat erklärt werden.

Bevorzugt ist der Duschvorhang dadurch gekennzeichnet, dass das Polykondensat als Verbindung der Gruppe c) Bis(1-hydroxy-2(1H)-pyridinthionato-O,S)-(T-4)-Zink aufweist. Metallorganische Komplexe und Verbindungen sind für Ihre Anti-Fouling Eigenschaften bekannt aber ob ihrer Toxizität kritisch zu sehen. Ähnlich wie bei den Benzimidazolen vermuten die Erfinder hier eine zusätzliche Fixierung dieser Wirkstoffkomponente innerhalb des Polykondensats durch die aromatische Struktur. Dadurch kann vorteilhaft eine hoch wirksame Komponente über das Polykondensat verankert und im Rahmen der Kondensation sogar anteilig mit in die Polykondensat-Struktur eingebunden werden. Die besondere Bewuchsbeständigkeit der so eingestellten Polykondensat-Beschichtung kann dadurch erklärt werden.

Bevorzugt ist der Duschvorhang dadurch gekennzeichnet, dass das Polykondensat ein Desodorant als Zusatzstoff aufweist. Ein Desodorant wirkt unangenehmen Gerüchen, wie sie im Bad über den Abfluss eindringen können, entgegen. Durch Zersetzung der dafür verantwortlichen Moleküle oder durch Überdecken der Gerüche mit angenehmen, stärker wahrnehmbaren Gerüchen, kann dem begegnet werden. Besonders bevorzugt wird das Desodorant als feucht aktivierbares Desodorant eingebracht, welches nur bei Kontakt mit Wasser gelöst und freigesetzt werden kann. In Kombination mit dem beanspruchten Polykondensat wird so ein Desodorant zugänglich, welches eine zuverlässige Langzeitwirkung ergänzend bereitstellt und vorteilhaft durch ausbleibenden 'frischen Geruch' eine Erschöpfung der Speicherkapazität des Langzeitdepots innerhalb des Polykondensats anzuzeigen vermag. Besonders bevorzugt ist der Duschvorhang zu diesem Zwecke mit einem heiß aktivierbaren Duftstoff-Träger als Zusatz ausgerüstet, der bei jeder Benutzung / jedem Duschen klar die Speicherkapazität des Polykondensats auf angenehme Weise indiziert.

Bevorzugt ist der Duschvorhang dadurch gekennzeichnet, dass das Polykondensat mindestens einen partikulären, anorganischen, optischen Aufheller ausgewählt aus der Gruppe bestehend aus Siliziumoxiden, Titandioxid, Zirkondioxid, Magnesiumoxid, Zinkoxid, Zinnoxid aufweist. Durch einen solchen Zusatz wird die UV-Beständigkeit des Gewebes erhöht und eine katalytische Umsetzung der keimtötenden Komponenten zusätzlich vermieden. Zudem wird besonders vorteilhaft ein optisch hoch weißer, nicht verbleichender Farbton eingestellt, welcher besonders bei bedruckten Duschvorhängen eine farbstabile und kontraststarke Grund- und Hintergrundfarbe des Gewebes schützt und auf lange Zeit sicherstellt.

Bevorzugt wird der beanspruchte Duschvorhang durch ein Verfahren erhalten, bei dem das Verfahren die Schritte umfasst a4) Einlegen eines Gewebes in ein Acrylat-Bad enthaltend mindestens ein aliphatisches Diol, eine Zink-organische Verbindung mit Hydroxylgruppe, Rest Hilfsstoffe, Zusätze und unvermeidbare Verunreinigungen, b) Entnahme des zumindest zu 80% getränkten Gewebes und c) abschließende Kondensation und Vernetzung.

Weitere Vorteile ergeben sich aus den Ausführungsbeispielen. Es versteht sich, dass die vorbeschriebenen Merkmale und Vorteile und nachfolgenden Ausführungsbeispiele nicht beschränkend aufzufassen sind. Vorteilhafte, zusätzliche Merkmale und zusätzliche Merkmalskombinationen, wie sie in der Beschreibung erläutert sind, können im Rahmen der unabhängigen Ansprüche im beanspruchten Gegenstand sowohl einzeln als auch abweichend kombiniert verwirklicht werden, ohne dass der Bereich der Erfindung verlassen würde.

### Detaillierte Erläuterung der Erfindung anhand von Ausführungsbeispielen

In besonders vorteilhafter Ausführungsform weist der Duschvorhang mit Antibewuchs-Ausrüstung ein auf seinem Gewebe verankertes, flüssiges Wasser abweisendes, gasdiffusionsoffen vernetztes, mehrkomponentiges Polykondensat auf. Das Polykondensat weist die Teilkondensate der Ausgangsstoffe a) Acrylat-Präpolymer in einer Menge von 1 bis 3 Gewichtsprozent bezogen auf das Grundgewicht des unbehandelten Duschvorhangs, b) 2-Methylpentan-2,4-diol in einer Menge von 0,05 bis 0,25 Gewichtsprozent bezogen auf das Grundgewicht des unbehandelten Duschvorhangs, c) Bis(1-hydroxy-2(1H)-pyridinthionato-O,S)-(T-4)-Zink in einer Menge von 0,1 bis 0,3 Gewichtsprozent bezogen auf das Grundgewicht des unbehandelten Duschvorhangs, 2-(4-Thiazolyl-)-1 H-benzimidazol als Zusatz in einer Menge von 0,1 bis 0,3 Gewichtsprozent bezogen auf das Grundgewicht des unbehandelten Duschvorhangs, sowie Rest Hilfsstoffe und unvermeidbare Verunreinigungen auf.

Der Duschvorhang wurde durch Tauchen in einem Ausrüstungsbad mit den vorgenannten Komponenten und abschließende Kondensation fertiggestellt. Nachfolgend wurde der Duschvorhang gewaschen, getrocknet. In besonders bevorzugter Ausführungsform erfolgte die Behandlung auf der Innenseite des Vorhangs mit einem abschließenden Trocknen / Aushärten um die 150°C +- 30°C.Der erhaltene Duschvorhang wurde auf seine keimtötende Wirkung untersucht:
Ein Segment des Gewebes wurde gemäß DIN EN ISO 20645 auf Agarplatten aufgebracht und jeweils eine Agarplatte wurde zum einen mit Staphylococcus aureus ATCC 6538 und zum anderen mit Escheria coli ATCC 11229 geimpft. Bei beiden Kulturen zeigte sich eine antibakterielle Wirkung in Form eines Hemmhofs, welcher um das jeweilige Segment sichtbar wurde: Staphylococcus aureus ATCC 6538 vermochte nur bis auf 6mm Distanz zum Segment einen Bewuchs auszubilden, während Escheria coli ATCC 11229 nur bis auf 1,5 mm Distanz zum Segment einen Bewuchs auf der Agarplatte ausbilden konnte. Ein gemäß DIN EN 1104 durchgeführter Test auf Wirkung gegen Aspergillus Niger DSM 1957 führte zu einem Hemmhof von nur 0,5 mm um die Schnittkanten des Gewebe-Segments herum. Die langsamen Durchtrittsgeschwindigkeiten der fungiziden, hydrophoben Bestandteile führen die Erfinder auf die kleinporige Natur des unter Wasserabspaltung gewonnenen Polyacrylat-Kondensats zurück, welche auch die Langzeitwirkung der aktiven Wirkstoffe zu erklären vermag. Eine antibakterielle/fungizide Wirkung des Duschvorhanges war somit nachgewiesen und konnte mit einem Modell konsistent dazu auf die mehrkomponentige Natur des Polykondensats auf Poly-Acrylat-Basis zurückgeführt werden.

In einem besonders vorteilhaften Verfahren wird ein erfindungsgemäßer Duschvorhang mit vorteilhafter Stabilität und verbessert gleichmäßiger, länger anhaltender Antibewuchs-Ausrüstung erhalten. Das vorteilhafte Verfahren umfasst die Schritte a0 zumindest außenseitiges Bedrucken des Gewebes, das Gewebe bestehend aus Polyester, a1) vollflächiges Aufbringen eines Primers zusammen mit einem vernetzbaren, Acrylat-kompatiblen Prepolymer - mit einer mittleren Prepolymer-Agglomeratgröße von 1 bis 50 Mikrometern - auf dem Gewebe, a2) Start der Vernetzungsreaktion im aufgebrachten Prepolymer, a3) Vorlegen des Acrylat-Bades, das Acrylat-Bad enthaltend

- 1 bis 5 Gewichtsteile Acrylsäure, - 1 bis 5 Gewichtsteile N-Butylacrylat, - 1 bis 5 Gewichtsteile Ethyl-Acrylat, - 1 bis 2 Gewichtsteile aliphatisches Diol, - 2 bis 4 Gewichtsteile Zink-organische Verbindung mit Hydroxylgruppe, - als Zusatz mindestens umfassend 2 bis 4 Gewichtsteile Benzimidazol-Verbindung, - als Hilfsstoff mindestens umfassend einen mit Temperaturen über 80°C deaktivierbaren Vernetzungs-Inhibitor, - Rest Wasser, optionale Hilfsstoffe, optionale Zusätze und unvermeidbare Verunreinigungen, a4) Einlegen des Gewebes in das Acrylat-Bad unter Unterbrechung der Vernetzungsreaktion im Prepolymer, b) Entnahme des zumindest zu 80% mit Acrylat-Bad getränkten Gewebes, c1) Vortrocknen und Beginn der Kondensation, c2) innenseitiges Erwärmen des Gewebes auf (120 +- 40) °C unter Deaktivierung des Inhibitors, nachfolgender Vernetzung und gleichzeitiger, abschließender Kondensation. Durch diese kombinierten Maßnahmen wird eine besonders stabil anhaftende und gleichmäßig gegen Bewuchs ausgerüstete Schicht erhalten, wie nachfolgend im Detail erläutert wird:

Zunächst wird ein Polyester-Gewebe zumindest außenseitig bedruckt. 'außenseitig' bezeichnet hierbei die Außenseite des Duschvorhangs, welche bei vorgezogenem Vorhang nach außen hin sichtbar ist, während die Innenseite des Vorhangs dem Duschraum zugewendet ist. Als Druckfarben können besonders vorteilhaft UV-beständige Pigmentfarben eingesetzt werden, welche über Jahre hinweg eine gute, stabile Farbintensität bereitstellen; besonders vorteilhaft wird die Bedruckung mit einem Zusatz an UV-Schutzstoff wir Titandioxid kombiniert, um die Bedruckung umfassend gegen Verblassen oder Verfärben langfristig zu schützen.

Ein aus Polyester gefertigter Duschvorhang zeichnet sich gegenüber Geweben auf Naturstoffbasis durch eine vorteilhafte Festigkeit und Maßbeständigkeit aus und ist den Naturstoffen insbesondere in seiner Verschleißbeständigkeit deutlich überlegen.

Das bedruckte Gewebe auf Polyester-Basis wird anschließend vollflächig mit einem Primer versehen. 'Primer' bezeichnet hierbei eine die Haftung einer Unterlage optimierende Komponente oder Mischung.

Bekannte Maßnahmen umfassen beispielsweise Aufbringung eines Haftvermittlers - umfassend Acrylate, Tanine, Brechweinstein, quaternäre Aminverbindungen, Silane, Polysilazane, Organosiloxane - in Kombination mit Maßnahmen wie dem Freilegen der zu beschichtenden Oberfläche durch Behandlung mit Säuren oder Laugen, Plasma- oder Coronabehandlung und Plasmaoxidation/-polymerisation.

Vorliegend wird ein vorteilhafter Haftvermittler in Form eines Organosiloxans mit einem Acrylat-kompatiblen, vernetzbaren Pre-Polymer aufgebracht. Als Organosiloxan bildete Methacrylsäureyltrimethoxysilan in Kombination mit einem - ggf. kohydrolysierten - Prepolymer besonders gleichmäßige, vernetzbare Schichten mit zuverlässiger Haftvermittlung aus. Beispiele für weitere Haftvermittler, kohydrolysierbare Mono- und Polymere und geeignete Reaktionsführungen zur Herstellung dieser Primer finden sich veranschaulichend in der DE 24 22 428, auf welche in vollem Umfang Bezug genommen wird; die in diesem Dokument beschriebenen Fotolacke erwiesen sich als überraschend gut geeignete Primer-Systeme für das vorliegende Verfahren. Besonders vorteilhaft wird ein Prepolymer auf Acrylatbasis verwendet, welches in seiner Reaktion gleichsinnig zum nachfolgenden Acrylat-Bad über die gleichen Inhibitoren einfacher steuerbar ist und keine zusätzlichen Additive erfordert.

Das Prepolymer weist eine mittlere Agglomeratgröße von 1 bis 50 Mikrometern auf. Mit Agglomeratgrößen im Mikrometerbereich konnten glatte und ebenmäßige Primer-Filme erzielt werden, welche nach kurzer, lichtinduzierter Vernetzung vorteilhaft gut auf dem Polyester anhafteten. Monomer-Kompositionen konnten bei kurzer Reaktionszeit nicht immer zuverlässig die für die Weiterverarbeitung notwendige Haftung bereitstellen, während Agglomerate im Millimeterbereich optisch deutlich sichtbar werden und das optische Erscheinungsbild der Bedruckung beeinträchtigen.

Die verankernde Vernetzung des Prepolymers kann vorteilhaft einfach photochemisch über einen Lichtblitz / geregelte, hellere Belichtung der gleichmäßig dünn verteilten Primer/Prepolymer-Schicht erfolgen. Die einsetzende, vernetzende Polymerisation verankert in kurzer Zeit das Prepolymer mit ausreichender Festigkeit.

Einbringen des mit einem Primer mit vernetzendem Prepolymer versehenen Gewebes in das vorbeschriebene Acrylat-Bad, welches - als Stabilisator der darin enthaltenen Acrylate - einen Vernetzungs-Inhibitor enthält, bringt die Vernetzung in der Primer/Prepolymer-Schicht zum erliegen. Die im Acrylat-Bad aufgenommene Ausrüstungs-Lösung kann daher ohne Hast gleichmäßig verteilt und aufgebracht werden, bis eine ebenmäßige, optisch saubere und einheitliche Schicht erzielt worden ist.

Diese Reaktionsführung bietet den besonderen Vorteil, dass bei der nachfolgenden Vortrocknung zunächst Wasser anteilig entfernt und eine erste Kondensation bewirkt wird. Mit einer Temperatur ab 80°C und höher wird der Vernetzungs-Inhibitor inaktiviert und die Vernetzung der Doppelbindungen der Acrylsäure-Verbindungen setzt wieder ein. Gleichzeitig bewirkt die parallele Kondensation von Hydroxylgruppen und Säuregruppen das Freisetzen und Austreten von Wasser. Die Erfinder gehen davon aus, dass das austretende Wasser die Ausrüstungs-Schicht während des Vernetzens gleichmäßig mit mikrofeinen Kanälen durchzieht, über welche später die gegen Bewuchs wirksamen Komponenten aktiv werden können; dies vermag die deutlich gleichmäßigere und über die Fläche länger anhaltende Wirksamkeit der vorteilhaft wie erläutert beschichteten Duschvorhänge zu erklären.

Durch den Acrylsäure-Anteil des Acrylat-Bades werden zusätzliche, kondensierbare Säuregruppen bereitgestellt, während die weiteren, enthaltenen Alkyl-Acrylate über ihre Alkylgruppen während der Vernetzung sterisch größere Molekülabstände notwendig machen und so eine offenere Struktur zusätzlich unterstützen.

Besonders vorteilhaft ist durch die unterbrochene Vernetzungsreaktion der Primer/Prepolymer-Schicht sichergestellt, dass die Vernetzung gleichmäßig Acrylate des Acrylat-Bads in die Primer/Prepolymer-Schicht mit einbindet, wodurch die an verfahrensgemäß hergestellten Mustern festgestellte, deutlich verbesserte Haftung und Beständigkeit gegen Abscheren und Abschälen bei wiederholtem Knicken des ausgerüsteten Gewebes erklärt werden kann.

Durch die innenseitige Erwärmung des Gewebes - beispielsweise mittels eine Heizstrahlers oder eines Heißluftstromes - wird eine außenseitige Bedruckung nur durch das Gewebe hindurch erwärmt; dadurch verläuft die Erwärmung außenseitig weniger drastisch, die Vernetzungsreaktion erfolgt langsamer und gleichmäßiger und das Druckbild bleibt in seiner optischen Wirkung vorteilhaft ohne jegliche Veränderung.

## Patentansprüche

1. Duschvorhang mit Antibewuchs-Ausrüstung, **dadurch gekennzeichnet, dass** dieser ein auf seinem Gewebe verankertes, flüssiges Wasser abweisendes, offen vernetztes, mehrkomponentiges Polykondensat aufweist, das Polykondensat aufweisend die per Acrylat-Bad eingebrachten und unter Wasserabspaltung in ein gemeinsames Gerüst eingebundenen Teilkondensate der Ausgangsstoffe
a) Acrylat-Präpolymer,
b) aliphatisches Diol und
c) Zink-organische Verbindung mit Hydroxylgruppe,
- Rest Hilfsstoffe, Zusätze und unvermeidbare Verunreinigungen,
wobei das Gewebe eine der Materialsorten Baumwolle, Wolle, Kunststoff und/oder Mischungen der Materialsorten aufweist.

2. Duschvorhang nach dem vorhergehenden Anspruch, **dadurch gekennzeichnet, dass** aliphatische Diole der Gruppe b) eine längste, unverzweigte Kette von 4 bis 8 C-Atomen aufweisen.

3. Duschvorhang nach einem der vorhergehenden Ansprüche, **dadurch gekennzeichnet, dass** das Polykondensat eine Benzimidazol-Verbindung als Zusatz aufweist.

4. Duschvorhang nach dem vorhergehenden Anspruch, **dadurch gekennzeichnet, dass** dass das Polykondensat 2-(4-Thiazolyl-)-1H-benzimidazol als Zusatz aufweist.

5. Duschvorhang nach einem der vorhergehenden Ansprüche, **dadurch gekennzeichnet, dass** das Polykondensat als Ausgangs-Diol der Gruppe b) 2-Methylpentan-2,4-diol als Diol aufweist.

6. Duschvorhang nach einem der vorhergehenden Ansprüche, **dadurch gekennzeichnet, dass** das Polykondensat als Verbindung der Gruppe c) Bis(1-hydroxy-2(1H)-pyridinthionato-O,S)-(T-4)-Zink aufweist.

7. Duschvorhang nach einem der vorhergehenden Ansprüche, **dadurch gekennzeichnet, dass** das Polykondensat ein Desodorant als Zusatzstoff aufweist.

8. Duschvorhang nach einem der vorhergehenden Ansprüche, **dadurch gekennzeichnet, dass** das Polykondensat einen heiß aktivierbaren Duftstoff-Träger als Zusatz aufweist.

9. Duschvorhang nach einem der vorhergehenden Ansprüche, **dadurch gekennzeichnet, dass** das Polykondensat mindestens einen partikulären, anorganischen optischen Aufheller ausgewählt aus der Gruppe bestehend aus Siliziumoxiden, Titandioxid, Zirkondioxid, Magnesiumoxid, Zinkoxid, Zinnoxid aufweist.

10. Duschvorhang nach einem der vorhergehenden Ansprüche, **dadurch gekennzeichnet, dass** dieser ein auf seinem Gewebe verankertes, flüssiges Wasser abweisendes, gasdiffusionsoffen vernetztes, mehrkomponentiges Polykondensat aufweist, das Polykondensat aufweisend die Teilkondensate der Ausgangsstoffe
a) Acrylat-Präpolymer in einer Menge von 1 bis 3 Gewichtsprozent bezogen auf das Grundgewicht des unbehandelten Duschvorhangs,
b) 2-Methylpentan-2,4-diol in einer Menge von 0,05 bis 0,25 Gewichtsprozent bezogen auf das Grundgewicht des unbehandelten Duschvorhangs
c) Bis(1-hydroxy-2(1H)-pyridinthionato-O,S)-(T-4)-Zink in einer Menge von 0,1 bis 0,3 Gewichtsprozent bezogen auf das Grundgewicht des unbehandelten Duschvorhangs,
2-(4-Thiazolyl-)-1 H-benzimidazol als Zusatz in einer Menge von 0,1 bis 0,3 Gewichtsprozent bezogen auf das Grundgewicht des unbehandelten Duschvorhangs, Rest Hilfsstoffe und unvermeidbare Verunreinigungen.

11. Verfahren zur Herstellung eines Dusch-Vorhangs nach einem der vorhergehenden Ansprüche, **dadurch gekennzeichnet, dass** das Verfahren die Schritte umfasst
a4) Einlegen eines Gewebes in ein Acrylat-Bad enthaltend mindestens ein aliphatisches Diol, eine Zink-organische Verbindung mit Hydroxylgruppe, Rest Hilfsstoffe, Zusätze und unvermeidbare Verunreinigungen
b) Entnahme des zumindest zu 80% getränkten Gewebes
c) abschließende Kondensation unter Wasserabspaltung und Vernetzung.

12. Verfahren zur Herstellung eines Duschvorhangs mit Antibewuchs-Ausrüstung, **dadurch gekennzeichnet, dass**
der hergestellte Duschvorhang ein auf seinem Gewebe verankertes, flüssiges Wasser abweisendes, offen vernetztes, mehrkomponentiges Polykondensat aufweist, das Gewebe eine der Materialsorten Baumwolle, Wolle, Kunststoff und/oder Mischungen der Materialsorten aufweist,
das Polykondensat aufweisend die per Acrylat-Bad eingebrachten und unter Wasserabspaltung in ein gemeinsames Gerüst eingebundenen Teilkondensate der Ausgangsstoffe Acrylat-Präpolymer, aliphatisches Diol und Zink-organische Verbindung mit Hydroxylgruppe, Rest Hilfsstoffe, Zusätze und unvermeidbare Verunreinigungen, wobei, dass das Verfahren die Schritte umfasst
a0) zumindest außenseitiges Bedrucken des Gewebes, das Gewebe bestehend aus Polyester,
a1) vollflächiges Aufbringen eines Primers zusammen mit einem vernetzbaren, Acrylat-kompatiblen Prepolymer, mit einer mittleren Prepolymer-Agglomeratgröße von 1 bis 50 Mikrometern, auf dem Gewebe
a2) Start der Vernetzungsreaktion in aufgebrachtem Prepolymer/Primer
a3) Vorlegen des Acrylat-Bades, das Acrylat-Bad enthaltend
- 1 bis 5 Gewichtsteile Acrylsäure,
- 1 bis 5 Gewichtsteile N-Butylacrylat,
- 1 bis 5 Gewichtsteile Ethyl-Acrylat,
- 1 bis 2 Gewichtsteile aliphatisches Diol,
- 2 bis 4 Gewichtsteile Zink-organische Verbindung mit Hydroxylgruppe,
- als Zusatz mindestens umfassend 2 bis 4 Gewichtsteile Benzimidazol-Verbindung,
- als Hilfsstoff mindestens umfassend einen mit Temperaturen über 80°C deaktivierbaren Vernetzungs-Inhibitor,
- Rest Wasser, optionale Hilfsstoffe, optionale Zusätze und unvermeidbare Verunreinigungen,
a4) Einlegen des Gewebes in das Acrylat-Bad unter Unterbrechung der Vernetzungsreaktion im Prepolymer/Primer,
b) Entnahme des zumindest zu 80% mit Acrylat-Bad getränkten Gewebes
c1) Vortrocknen und Beginn der Kondensation
c2) innenseitiges Erwärmen des Gewebes auf (120 +- 40) °C unter Deaktivierung des Inhibitors, nachfolgender Vernetzung und gleichzeitiger, abschließender Kondensation.

## Claims

1. Shower curtain with antifouling treatment, **characterized in that** this has, anchored on its fabric, a liquid-water-repellant, open-crosslinked, multicomponent polycondensate which comprises the partial condensates, introduced by way of an acrylate bath, and bonded together into a structure with elimination of water, of the following starting materials:
a) acrylate prepolymer,
b) aliphatic diol, and
c) organozinc compound having hydroxy group,
- remainder being auxiliaries, additions, and unavoidable contaminants,
where the fabric comprises one of the following types of material: cotton, wool, plastic, and/or mixtures of these types of material.

2. Shower curtain according to the preceding claim, **characterized in that** aliphatic diols of the group b) have a longest, unbranched chain of from 4 to 8 C atoms.

3. Shower curtain according to any of the preceding claims, **characterized in that** the polycondensate comprises a benzimidazole compound as addition.

4. Shower curtain according to the preceding claim, **characterized in that** the polycondensate comprises 2-(4-thiazolyl)-1H-benzimidazole as addition.

5. Shower curtain according to any of the preceding claims, **characterized in that** the polycondensate comprises, as diol, 2-methylpentan-2,4-diol as starting diol of the group b).

6. Shower curtain according to any of the preceding claims, **characterized in that** the polycondensate comprises bis(1-hydroxy-2(1H)-pyridinethionato-O,S)-(T-4)-zinc as compound of the group c).

7. Shower curtain according to any of the preceding claims, **characterized in that** the polycondensate comprises, as additional substance, a deodorant.

8. Shower curtain according to any of the preceding claims, **characterized in that** the polycondensate comprises, as addition, a heat-activatable fragrance carrier.

9. Shower curtain according to any of the preceding claims, **characterized in that** the polycondensate comprises at least one particulate, inorganic optical brightener selected from the group consisting of silicon oxides, titanium dioxide, zirconium dioxide, magnesium oxide, zinc oxide, tin oxide.

10. Shower curtain according to any of the preceding claims, **characterized in that** this has, anchored on its fabric, a liquid-water-repellent, multicomponent polycondensate which has been crosslinked in a manner that permits gas diffusion and which comprises the partial condensates of the following starting materials:
a) a quantity of from 1 to 3 percent by weight, based on the underlying weight of the untreated shower curtain, of acrylate prepolymer,
b) a quantity of from 0.05 to 0.25 percent by weight, based on the underlying weight of the untreated shower curtain, of 2-methylpentane-2,4-diol,
c) a quantity of from 0.1 to 0.3 percent by weight, based on the underlying weight of the untreated shower curtain, of bis(1-hydroxy-2(1H)-pyridinethionato-O,S)-(T-4)-zinc,
a quantity of from 0.1 to 0.3 percent by weight, based on the underlying weight of the untreated shower curtain, of 2-(4-thiazolyl)-1H-benzimidazole,
remainder being auxiliaries and unavoidable contaminants.

11. Process for the production of a shower curtain according to any of the preceding claims, **characterized in that** the process comprises the following steps:
a4) placement of a fabric into an acrylate bath comprising at least one aliphatic diol, one organozinc compound having hydroxy group, remainder being auxiliaries, additions, and unavoidable contaminants,
b) removal of the least 80% saturated fabric,
c) final condensation with elimination of water and crosslinking.

12. Process for the production of a shower curtain with antifouling treatment, **characterized in that** the shower curtain produced has, anchored on its fabric, a liquid-water-repellant, open-crosslinked, multicomponent polycondensate,
the fabric comprises one of the following types of material, cotton, wool, plastic, and/or mixtures of these types of material, the polycondensate comprises the partial condensates, introduced by way of an acrylate bath, and bonded together into a structure with elimination of water, of the following starting materials: acrylate prepolymer, aliphatic diol, and organozinc compound having hydroxy group, remainder being auxiliaries, additions, and unavoidable contaminants, where the process comprises the following steps:
a0) at least printing of the external side of the fabric, which is composed of polyester,
a1) full-surface application, on the fabric, of a primer together with a crosslinkable, acrylate-compatible prepolymer with an average agglomerate size of from 1 to 50 micrometers, a2) start of the crosslinking reaction in prepolymer/primer applied,
a3) provision of the acrylate bath which comprises
- from 1 to 5 parts by weight of acrylic acid,
- from 1 to 5 parts by weight of n-butyl acrylate,
- from 1 to 5 parts by weight of ethyl acrylate,
- from 1 to 2 parts by weight of aliphatic diol,
- from 2 to 4 parts by weight of organozinc compound having hydroxy group,
- addition at least comprising from 2 to 4 parts by weight of benzimidazole compound,
- auxiliary at least comprising a crosslinking inhibitor deactivatable by temperatures above 80°C,
- remainder being water, optional auxiliaries, optional additions, and unavoidable contaminants,
a4) placement of the fabric into the acrylate bath with interruption of the crosslinking reaction in the prepolymer/primer,
b) removal of the at least 80% acrylate-bath-saturated fabric,
c1) predrying and beginning of condensation,
c2) heating of the internal side of the fabric to (120 +- 40)°C with deactivation of the inhibitor, then with crosslinking and with simultaneous, final condensation.

## Revendications

1. Rideau de douche à protection anti-salissures, **caractérisé en ce que** celui-ci comprend un polycondensat multicomposant ancré sur son tissu, répulsif pour l'eau liquide, réticulé ouvert, le polycondensat comprenant les condensats partiels introduits par un bain d'acrylate et reliés dans un squelette commun avec clivage d'eau, des matières premières suivantes .
a) un prépolymère d'acrylate,
b) un diol aliphatique et
c) un composé organique de zinc contenant un groupe hydroxyle,
- le reste étant des adjuvants, des additifs et des impuretés inévitables,
le tissu comprenant un des types de matériaux suivants : coton, laine, plastique et/ou des mélanges des types de matériaux.

2. Rideau de douche selon la revendication précédente, **caractérisé en ce que** les diols aliphatiques du groupe b) présentent une chaîne non ramifiée la plus longue de 4 à 8 atomes C.

3. Rideau de douche selon l'une quelconque des revendications précédentes, **caractérisé en ce que** le polycondensat comprend un composé de benzimidazole en tant qu'additif.

4. Rideau de douche selon la revendication précédente, **caractérisé en ce que** le polycondensat comprend du 2-(4-thiazolyl-)-1H-benzimidazole en tant qu'additif.

5. Rideau de douche selon l'une quelconque des revendications précédentes, **caractérisé en ce que** le polycondensat comprend en tant que diol de départ du groupe b) du 2-méthylpentane-2,4-diol en tant que diol.

6. Rideau de douche selon l'une quelconque des revendications précédentes, **caractérisé en ce que** le polycondensat comprend en tant que composé du groupe c) du bis(1-hydroxy-2(1H)-pyridinethionato-O,S)-(T-4)-zinc.

7. Rideau de douche selon l'une quelconque des revendications précédentes, **caractérisé en ce que** le polycondensat comprend un désodorisant en tant qu'additif.

8. Rideau de douche selon l'une quelconque des revendications précédentes, **caractérisé en ce que** le polycondensat comprend un support de parfum thermoactivable en tant qu'additif.

9. Rideau de douche selon l'une quelconque des revendications précédentes, **caractérisé en ce que** le polycondensat comprend au moins un azurant optique inorganique particulaire choisi dans le groupe constitué par les oxydes de silicium, le dioxyde de titane, le dioxyde de zirconium, l'oxyde de magnésium, l'oxyde de zinc, l'oxyde d'étain.

10. Rideau de douche selon l'une quelconque des revendications précédentes, **caractérisé en ce que** celui-ci comprend un polycondensat multicomposant, ancré sur son tissu, répulsif pour l'eau liquide, réticulé ouvert à la diffusion de gaz, le polycondensat comprenant les condensats partiels des matières premières suivantes :
a) un prépolymère d'acrylate en une quantité de 1 à 3 pour cent en poids par rapport au poids de base du rideau de douche non traité,
b) du 2-méthylpentane-2,4-diol en une quantité de 0,05 à 0,25 pour cent en poids par rapport au poids de base du rideau de douche non traité,
c) du bis(1-hydroxy-2(1H)-pyridinethionato-O,S)-(T-4)-zinc en une quantité de 0,1 à 0,3 pour cent en poids par rapport au poids de base du rideau de douche non traité,
du 2-(4-thiazolyl-)-1H-benzimidazole en tant qu'additif en une quantité de 0,1 à 0,3 pour cent en poids par rapport au poids de base du rideau de douche non traité,
le reste étant des adjuvants et des impuretés inévitables.

11. Procédé de fabrication d'un rideau de douche selon l'une quelconque des revendications précédentes, **caractérisé en ce que** le procédé comprend les étapes suivantes :
a4) la mise en place d'un tissu dans un bain d'acrylate contenant au moins un diol aliphatique, un composé organique de zinc contenant un groupe hydroxyle, le reste étant des adjuvants, des additifs et des impuretés inévitables,
b) l'extraction du tissu imprégné à au moins 80 %, puis
c) la condensation avec clivage d'eau et réticulation.

12. Procédé de fabrication d'un rideau de douche à protection anti-salissures, **caractérisé en ce que** le rideau de douche fabriqué comprend un polycondensat multicomposant ancré sur son tissu, répulsif pour l'eau liquide, réticulé ouvert,
le tissu comprend un des types de matériaux suivantes :
coton, laine, plastique et/ou des mélanges des types de matériaux,
le polycondensat comprenant les condensats partiels introduits par un bain d'acrylate et reliés dans un squelette commun avec clivage d'eau, des matières premières suivantes : prépolymère d'acrylate, diol aliphatique et composé organique de zinc contenant un groupe hydroxyle, le reste étant des adjuvants, des additifs et des impuretés inévitables,
le procédé comprenant les étapes suivantes :
a0) l'impression au moins du côté extérieur du tissu, le tissu étant constitué de polyester,
a1) l'application sur l'ensemble de la surface d'une amorce conjointement avec un prépolymère réticulable compatible avec les acrylates, ayant une taille d'agglomérat de prépolymère moyenne de 1 à 50 micromètres, sur le tissu,
a2) le début de la réaction de réticulation dans le prépolymère/l'amorce appliqué,
a3) la mise à disposition du bain d'acrylate, le bain d'acrylate contenant :
- 1 à 5 parties en poids d'acide acrylique,
- 1 à 5 parties en poids d'acrylate de N-butyle,
- 1 à 5 parties en poids d'acrylate d'éthyle,
- 1 à 2 parties en poids d'un diol aliphatique,
- 2 à 4 parties en poids d'un composé organique de zinc contenant un groupe hydroxyle,
- en tant qu'additif, comprenant au moins 2 à 4 parties en poids d'un composé de benzimidazole,
- en tant qu'adjuvant, comprenant au moins inhibiteur de réticulation désactivable à des températures supérieures à 80 °C,
- le reste étant de l'eau, des adjuvants éventuels, des additifs éventuels et des impuretés inévitables,
a4) la mise en place du tissu dans le bain d'acrylate avec interruption de la réaction de réticulation dans le prépolymère/l'amorce,
b) l'extraction du tissu imprégné à au moins 80 % avec le bain d'acrylate,
c1) le pré-séchage et le début de la condensation,
c2) le chauffage du côté intérieur du tissu à (120 ± 40) °C avec désactivation de l'inhibiteur, puis la réticulation et enfin la condensation simultanée.
